(19) **Europäisches Patentamt**

**European Patent Office**

**Office européen des brevets**

(11) **EP 0 951 308 B1**

(12) **EUROPÄISCHE PATENTSCHRIFT**

(45) Veröffentlichungstag und Bekanntmachung des Hinweises auf die Patenterteilung:
**24.05.2000 Patentblatt 2000/21**

(51) Int Cl.$^7$: **A61M 5/172**

(21) Anmeldenummer: **97951973.3**

(86) Internationale Anmeldenummer:
**PCT/EP97/06574**

(22) Anmeldetag: **25.11.1997**

(87) Internationale Veröffentlichungsnummer:
**WO 98/24496 (11.06.1998 Gazette 1998/23)**

(54) **MEDIKAMENTEN-DOSIERSYSTEM**

MEDICAMENT DOSING SYSTEM

SYSTEME DE DOSAGE DE MEDICAMENTS

(84) Benannte Vertragsstaaten:
**BE DE FR GB IT NL**

(30) Priorität: **03.12.1996 DE 19650115**

(43) Veröffentlichungstag der Anmeldung:
**27.10.1999 Patentblatt 1999/43**

(73) Patentinhaber: **FRAUNHOFER-GESELLSCHAFT ZUR FÖRDERUNG DER ANGEWANDTEN FORSCHUNG E.V.**
**80636 München (DE)**

(72) Erfinder:
• **WOIAS, Peter**
  **D-81735 München (DE)**
• **RICHTER, Martin**
  **D-81677 München (DE)**

(74) Vertreter: **Schoppe, Fritz, Dipl.-Ing.**
**Schoppe, Zimmermann & Stöckeler**
**Patentanwälte**
**Postfach 71 08 67**
**81458 München (DE)**

(56) Entgegenhaltungen:
**WO-A-86/05993          WO-A-96/32975**

**Beschreibung**

Die vorliegende Erfindung bezieht sich auf ein Medikamenten-Dosiersystem und insbesondere auf ein Medikamenten-Dosiersystem, das nach dem Überdruckprinzip arbeitet.

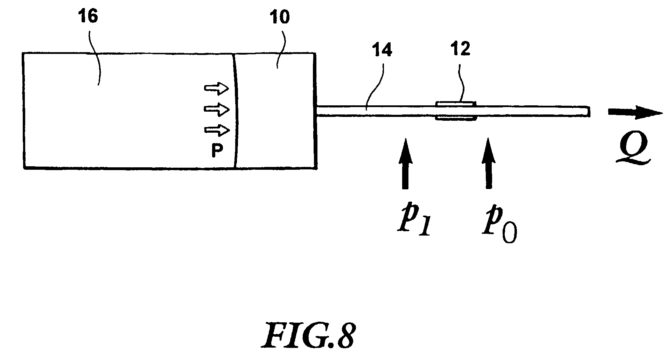

Eine Medikamenten-Dosierung wird in jüngerer Zeit vor allem durch Dosiersysteme, die nach dem Überdruckprinzip arbeiten, durchgeführt. Eine schematische Darstellung zur Veranschaulichung des Überdruckprinzips ist in Fig. 8 dargestellt. Derartige Systeme bestehen aus einem Fluidreservoir 10 und einem Flußwiderstand 12, der beispielsweise an oder in einer Fluidleitung 14, die mit dem Fluidreservoir 10 verbunden ist, angeordnet ist. Eine Druckgebereinrichtung 16 dient dazu, das in dem Fluidreservoir 10 befindliche flüssige Medikament mit einem Druck zu beaufschlagen. Die Druckgebereinrichtung erzeugt einen Druck p, wodurch das Fluidreservoir 10 mit einem bestimmtem Überdruck $p_1$ bezogen auf den Druck $p_0$ am Auslaß des Flußwiderstandes 12 beaufschlagt wird. Der Druck $p_1$ entspricht dabei im wesentlichen dem durch die Druckgebereinrichtung 16 erzeugten Druck p. Im Betrieb entsteht durch den am Flußwiderstand 12 anliegenden Differenzdruck ein Fluß Q.

Für einen kreisförmigen Querschnitt des Flußwiderstands kann die Größe des Flusses Q nach dem bekannten Gesetz von Hagen-Poiseuille berechnet werden:

$$Q = \frac{\pi \cdot \Delta p \cdot R^4}{8\eta L}$$

Die Flußrate Q wird von folgenden Einflußgrößen bestimmt:

- Der Viskosität η des Mediums,
- dem effektiven Strömungsquerschnitt $\pi R^4/8$ und der Länge L des Flußwiderstandes,
- dem Differenzdruck Δp zwischen dem Einlaß und dem Auslaß des Flußwiderstandes, und
- der Temperatur als indirekter Einflußgröße, beispielsweise über die temperaturabhängige Viskosität des Fluids.

Für andere Strömungsquerschnitte können analoge Gesetzmäßigkeiten bestimmt werden, die sich von der in der obigen Gleichung genannten Gesetzmäßigkeit im wesentlichen in der Berücksichtigung des effektiven Strömungsquerschnittes des Flußwiderstandes unterscheiden. Derartige analoge Gesetzmäßigkeiten beispielsweise für mikromechanisch gefertigte Flußwiderstände sind in "Micro Channels for Applications in Liquid Dosing and Flow Rate Measurement M. Richter, P.Woias, D.Weiß, Proceedings of Euro Sensors X, 8. bis 11. September 1996, Leuven, Belgien, Band 4, Seiten 1297 bis 1300, beschrieben.

Die technische Ausgestaltung bestehender Dosiersysteme ist sehr unterschiedlich, und benutzt in vielfältiger Kombination Mechanismen wie mechanische Systeme, beispielsweise Federdrucksysteme, elektrochchemische Systeme, beispielsweise Elektrolysezellen, thermopneumatische Systeme, beispielsweise den Verdampfungsdruck einer leicht flüchtigen Substanz, und die Schwerkraft. Als Flußwiderstand werden üblicherweise Kunststoffkapillare, Glaskapillare und Metallkapillare eingesetzt.

Gemäß der oben dargelegten Gleichung beeinflußt bei einem kreisförmigen Querschnitt des Flußwiderstandes der Radius R durch den Term $R^4$ mit vierter Potenz die Flußrate Q. Dies bedeutet, daß zur Erzielung einer exakten Dosierung Flußwiderstände mit einer hohen geometrischen Genauigkeit realisiert werden müssen. Eine derartige Genauigkeit ist nur durch einen vergleichsweise hohen technischen Aufwand möglich. Einfache Systeme, die Kunststoffkapillare aufweisen, sind ferner nachteilig dahingehend, daß sich die Kapillare abhängig vom anliegenden Druck dehnt, wodurch die Genauigkeit der Dosierung abnimmt.

Es sind ferner vergleichbare mikromechanische Ausführungen zur Glukosemessung mittels einer Mikrodialyse bekannt. Bei einer derartigen mikromechanischen Ausführung ist eine Mikrokapillare zur Flußeinstellung zusammen mit Glucose-Sensoren auf einem Silizium-Chip realisiert. Dieser bekannte Aufbau dient allerdings nicht zur Medikamentendosierung, sondern nutzt das oben beschriebene Überdruckprinzip lediglich zur Flußrateneinstellung des Trägermediums für die Mikrodialyse.

Ein weiteres bekanntes, implementierbares mikromechanisches Dosiersystem verwendet ein Lösungsmittel-Reservoir als Konstantdruckgeber und ein Array mikromechanisch realisierter Flußwiderstände zur Flußrateneinstellung. Die gesamte Fließstrecke wird durch An- bzw. Ab-Koppeln einzelner Mikro-Flußwiderstände über jeweils zugeordnete Mikroventile in ihrer Geometrie variiert, wodurch eine stufenweise geschaltete Variation der Dosierrate erreicht wird. Zur Messung des Flusses werden dabei Drucksensoren an verschiedenen Stellen des Arrays verwendet. Ein solches System mit einem Array aus Mikro-Flußrestriktionen erlaubt keine stufenlose Einstellung der Dosierrate, wobei ferner der technische Aufwand sehr hoch ist, da mehrere Mikroventile und Drucksensoren erforderlich sind, was aus Kostengründen nur einen beschränkten Einsatzbereich zuläßt.

Die Mehrheit der bekannten Dosiersysteme ist von außen nicht beeinflußbar, d.h. die Flußrate kann im Betrieb nicht variiert werden, was beispielsweise notwendig ist, um eine cirkadiane Rhythmik bei der Dosierung automatisch einzuhalten. Ferner wird bei den bekannten Dosiersystemen der Einfluß der Temperatur auf die Viskosität des Fluids und damit auf die Dosierrate in der Regel nicht kompensiert. Dies kann insbesondere bei tragbaren Dosiersystemen zu beträchtlichen Dosierfehlern führen. Sollen sehr geringe Flußraten eingestellt werden, µl/min bis pl/min, sind Flußwiderstände mit effektiven Querschnittabmessungen im µm-Bereich erforderlich. Derartige Querschnittabmessungen können mit konventionellen Techniken nicht hergestellt werden. Bekannte Dosiersysteme, die keine aufwendigen Bauteile als Einwegteile verwenden, ermöglichen keine stufenlose Einstellung der Dosierrate.

Die DE-A-19501691 beschreibt ein nicht invasives System zur Fluidstromüberwachung, bei dem die Infusionsrate durch eine gepulste Ansteuerung einer Fluidförderpumpe eingestellt wird. Die Temperatur des geförderten Fluids wird dabei mit einem Temperaturfühler überwacht.

In der DE-A-3827444 sind ein Verfahren und eine Vorrichtung zum Nachweis einer Flüssigkeitsströmung in einer Leitung beschrieben. Die bekannte Vorrichtung weist eine an der Leitung angebrachte Heizeinrichtung und zwei Temperaturfühler auf, die stromaufwärts und stromabwärts von der Heizeinrichtung an der Leitung angebracht sind. Die an den Temperaturfühlern gemessenen Temperaturen dienen dazu, Aussagen über das Vorliegen einer Strömung, die Strömungsrichtung sowie die Strömungsgeschwindigkeit zu treffen.

Die Aufgabe der vorliegenden Erfindung besteht darin, ein Medikamenten-Dosiersystem zu schaffen, das eine stufenlose Einstellung der Dosierrate ermöglicht und ferner keine aufwendigen Einwegteile aufweist.

Diese Aufgabe wird durch Medikamenten-Dosiersysteme gemäß Anspruch 1 und Anspruch 2 gelöst.

Die vorliegende Erfindung schafft ein Medikamenten-Dosiersystem, das aus einer auswechselbaren Einheit und einer dauerhaften Einheit besteht. Die auswechselbare Einheit weist ein Fluidreservoir zur Aufnahme eines unter Druck setzbaren, flüssigen Medikaments, einen Temperatursensor zum Erfassen der Temperatur des flüssigen Medikaments, einen mit einem Flußwiderstand versehenen, fluidmäßig mit dem Fluidreservoir verbundenen Fluidkanal und eine mit dem Fluidkanal verbundene Schlaucheinrichtung auf. Die dauerhafte Einrichtung weist eine Quetschventilvorrichtung zum Zusammenquetschen der Schlaucheinrichtung und eine Steuereinrichtung, die mit dem Temperatursensor und der Quetschventilvorrichtung gekoppelt ist, auf, um eine Flußrate des flüssigen Medikaments durch ein getaktetes Betätigen der Quetschventilvorrichtung abhängig von der erfaßten Temperatur zu steuern. Alternativ kann der Temperatursensor zum Erfassen der Temperatur des flüssigen Medikaments auch in der dauerhaften Einrichtung angeordnet sein.

Das in dem Fluidreservoir befindliche flüssige Medikament wird vorzugsweise durch einen Konstantdruckgeber mit einem Druck beaufschlagt, wobei das erfindungsgemäße Medikamenten-Dosiersystem ferner zumindest einen Drucksensor aufweisen kann um den Druck des flüssigen Medikaments vor dem Flußwiderstand zu erfassen. Ferner kann das erfindungsgemäße Medikament-Dosiersystem einen weiteren Drucksensor nach dem Flußwiderstand aufweisen, wobei die Steuereinrichtung die Flußrate des flüssigen Medikaments abhängig von der erfaßten Temperatur und der Differenz der erfaßten Drücke steuert.

Das erfindungsgemäße Dosiersystem arbeitet nach dem Überdruckprinzip, und verwendet vorzugsweise einen mikromechanisch gefertigten Flußwiderstand und bietet Möglichkeiten zur externen Beeinflussung der Dosierrate über die Steuervorrichtung und zur Kompensation von Temperatureffekten.

Bei dem erfindungsgemäßen Medikamenten-Dosiersystem ist ein Quetschventil, das von der Steuereinrichtung getaktet gesteuert wird, verwendet, um einen Durchfluß durch den Schlauch zu ermöglichen oder zu verhindern. Dieses Quetschventil nimmt den Schlauch von außen in Eingriff und quetscht ihn zusammen, um einen Durchfluß des flüssigen Medikaments zu verhindern. Dadurch kommt das Ventil nicht mit dem Medikament in Berührung. Somit kann das Ventil ein Teil der dauerhaften Vorrichtung sein. Der Einwegteil der erfindungsgemäßen Medikamenten-Dosiervorrichtung umfaßt somit nur die vorzugsweise mikromechanisch gefertigten Bauteile des Fluidreservoirs, und des mit dem Fluidreservoir verbundenen, einen Flußwiderstand aufweisenden Fluidkanals und die optional integrierten Drucksensoren, sowie den Schlauch, der mit dem Fluidkanal verbunden ist, Bei einem Ausführungsbeispiel weist das Einwegteil ferner den Temperatursensor auf. Die vorliegende Erfindung ist somit gegenüber den bekannten Dosiersystemen vorteilhaft dahingehend, daß dieselbe keine teuren Einwegteile aufweist.

Das erfindungsgemäße Medikamenten-Dosiersystem ermöglicht eine stufenlose Einstellung der Dosierrate, wobei im Vergleich zum bisherigen Stand der Technik keine kostenmäßig aufwendigeren Einwegteile verwendet werden. Das erfindungsgemäße System beruht auf dem bereits bekannten Prinzip der Überdruckdosierung beispielsweise unter Verwendung von Konstant-Druckgebern und erlaubt somit die Weiterbildung bereits bestehender Systeme zur Erzielung verbesserter Eigenschaften. Der Aufbau der verwendeten Komponenten erlaubt durch günstigere Herstellungskosten bei verbesserter Funktionalität, beispielsweise geringere Dosierraten, eine höhere Herstellungsgenauigkeit und somit ein geringerer Aufwand, sowohl den Einwegbetrieb als auch dadurch, daß im sterilen Bereich nur eine Mikromechanik zum Einsatz kommt, die mehrfache Verwendung mit bedarfsmäßiger Zwischensterilisation. Das erfindungsgemäße System kann somit sowohl für den reinen Heim-Betrieb (Home-Care-Betrieb), Einwegbetrieb, als auch für den Klinik-Betrieb mit mehrfacher Verwendung verwendet werden.

Bevorzugte Ausführungsbeispiele der vorliegenden Erfindung werden nachfolgend bezugnehmend auf die beiliegenden Zeichnungen näher erläutert. Es zeigen:

Fig. 1      eine schematische Darstellung eines Medikamenten-Dosiersystems gemäß der vorliegenden Erfindung;

Fig. 2      Diagramme zur Veranschaulichung der Funktionsweise des erfindungsgemäßen Medikamenten-Dosiersystems;

Fig. 3A bis 3C      schematische Darstellungen eines mikromechanisch gefertigten Flußwiderstandes, der in dem Medikamenten-Dosiersystem gemäß der vorliegenden Erfindung verwendbar ist;

Fig. 4A und 4B      eine Schnittansicht bzw. eine Draufsicht eines Ausführungsbeispiels eines mikromechanisch gefertigten Flußwiderstandes mit integrierten Drucksensoren.

Fig. 5      eine Schnittansicht eines weiteren Ausführungsbeispiels eines mikromechanisch gefertigten Flußwiderstandes mit integrierten Drucksensoren; und

Fig. 6      eine schematische Darstellung eines sogenannten Luer-Systems zur fluidischen Ankopplung von mikromechanisch gefertigten Fluidsystemen;

Fig. 7A und 7B      jeweils eine Querschnittansicht und eine Draufsicht weiterer Ausführungsbeispiele von mikromechanisch gefertigten Flußwiderständen; und

Fig. 8      eine schematische Darstellung zur Veranschaulichung des Überdruckprinzips.

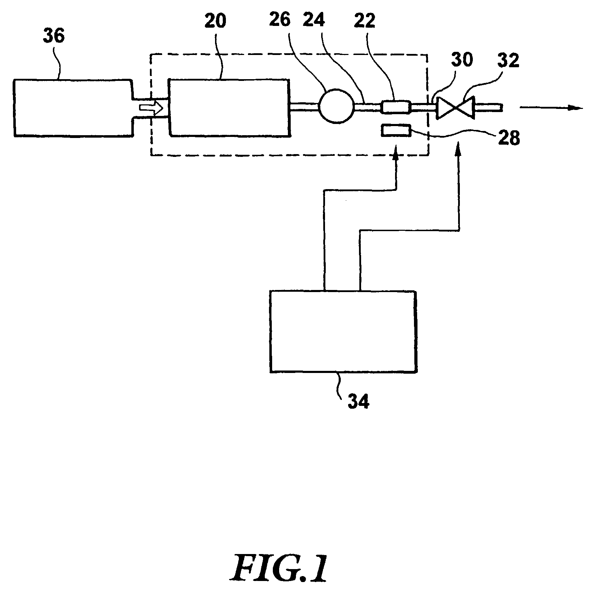

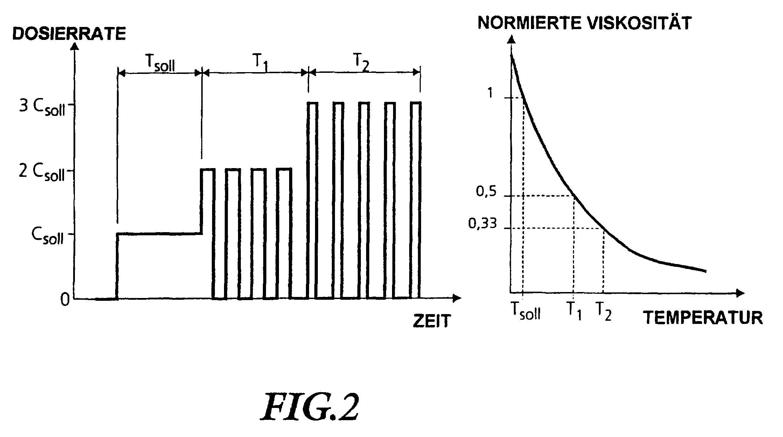

Bezugnehmend auf die Fig. 1 und 2 wird nachfolgend ein bevorzugtes Ausführungsbeispiel des erfindungsgemäßen Medikamenten-Dosiersystems beschrieben. Das System weist ein Fluidreservoir 20 und einen Flußwiderstand 22, der mit dem Fluidreservoir 20 fluidmäßig verbunden ist, beispielsweise über eine Fluidleitung 24, auf. Bei dem dargestellten Ausführungsbeispiel ist optional zwischen dem Fluidreservoir 20 und dem Fluidwiderstand 22 ein Filter 26 angeordnet. Das Filter 26 kann zum Filtern von Luftblasen und/oder zum Filtern von Bakterien dienen. Ein Temperatursensor 28 zum Erfassen der Temperatur des flüssigen Medikaments ist vorzugsweise im Bereich des Flußwiderstandes 22 angeordnet. Die in Fig. 1 innerhalb der gestrichelten Umrandung dargestellten Bauteile stellen die austauschbaren Teile des erfindungsgemäßen Medikamenten-Dosiersystems, d.h. die Einwegteile, dar. Alternativ kann der Temperatursensor derart in der Medikamenten-Dosiervorrichtung angeordnet sein, daß er die Temperatur des flüssigen Medikaments erfassen kann, wobei er jedoch nicht dem Einwegteil zugeordnet ist.

Der Flußwiderstand 22 ist am Auslaß mit einer flexiblen Schlaucheinrichtung 30 fluidmäßig verbunden. Die Schlaucheinrichtung 30 kann durch ein Quetschventil 32 durch Betätigen desselben zusammengequetscht werden, und ist, wenn das Quetschventil 32 nicht betätigt ist, für einen Fluiddurchfluß offen. Mit dem Temperatursensor 28 und dem Quetschventil 32 ist eine Steuereinheit 34 elektrisch gekoppelt.

In Fig. 1 ist ferner schematisch ein Druckgeber 36 dargestellt, der ein in dem Fluidreservoir 20 befindliches flüssiges Medikament mit einem Druck beaufschlagt. Der Druckgeber 36 kann beispielsweise ein Konstant-Druckgeber in einer beliebigen bekannten Ausführung sein. Als Druckgeber können beispielsweise mechanische Systeme, elektrochemische Systeme oder thermopneumatische Systeme verwendet sein. Ferner kann auch die Schwerkraft verwendet werden, um das flüssige Medikament unter Druck zu setzen. Alternativ kann der Druckgeber durch die Umrandung des Fluidreservoirs gebildet sein, wenn diese elastisch, sich zusammenziehend, ausgebildet ist, oder durch ein Gas, das neben dem flüssigen Medikament in dem Fluidreservoir vorliegt und das flüssige Medikament somit unter Druck setzt.

Die Steuereinrichtung 34 weist vorzugsweise eine Datenschnittstelle, einen Mikrocontroller sowie eine Anzeigevorrichtung, beispielsweise einen Bildschirm, und eine Eingabevorrichtung, beispielsweise eine Tastatur, auf.

Wie oben bezüglich des Standes der Technik erläutert wurde, hängt die Flußrate durch einen Flußwiderstand von der Viskosität des Mediums, dem effektiven Strömungsquerschnitt und der Länge des Flußwiderstandes und dem Differenzdruck zwischen dem Einlaß und dem Auslaß des Flußwiderstandes ab. Die Abhängigkeit des Flusses von diesen Größen für bestimmte Strömungsquerschnitte ist in der oben erwähnten Schrift "Micro Channels for Applications in Liquid Dosing and Flow Rate Measurement" erläutert.

Bei einem gegebenen Querschnitt des Flußwiderstandes hängt die Flußrate somit von dem Differenzdruck und der Temperatur des Mediums ab, da die Viskosität des Mediums stark mit der Temperatur desselben schwankt. Bei einem gegebenen konstanten Druck, der von dem Druckgeber 36 geliefert wird, hängt die Flußrate somit ausschließlich von der Temperatur des Mediums ab. Die Steuereinheit 34 erfaßt diese Temperatur und taktet das Steuerventil derart, daß sich für die erfaßte Temperatur eine gewünschte Flußrate ergibt.

Der Mikroflußwiderstand ist vorzugsweise derart dimensioniert, daß bei der niedrigsten denkbaren Betriebstemperatur $T_{soll}$ der gewünschte Soll-Fluß im kontinuierlichen Betrieb, d.h. bei einer dauerhaften Öffnung des Quetschventils 32, eingehalten wird. Sobald die Temperatur diesen Wert übersteigt, erfolgt die Dosierung nicht mehr kontinuierlich, sondern mit Hilfe des Quetschventils 32 im EIN/AUS-Wechselbetrieb.

Abhängig von der erfaßten Temperatur und einer vorzugsweise in einem Speicher der Steuereinrichtung 34 gespeicherten Viskositäts-Temperaturkurve sowie anhand des oben erläuterten Zusammenhangs zwischen der Flußrate und den physikalischen Parametern des Flußwiderstandes und des Mediums bestimmt die Steuereinrichtung 34 das Puls-Pausenverhältnis derart, daß die effektive Dosierrate, d.h. das Produkt aus Flußrate und Tastverhältnis, konstant gehalten wird.

Eine schematische Darstellung dieses Steuerverfahrens ist in Fig. 2 dargestellt. Im rechten Teil von Fig. 2 ist die normierte Viskosität eines zu transportierenden Mediums über der Temperatur dargestellt. Bei der Temperatur $T_{soll}$ beträgt die normierte Viskosität 1, während die Viskosität mit zunehmender Temperatur abnimmt, bei $T_1$ auf 0,5 und bei $T_2$ auf 0,33.

Wie oben erläutert wurde, ist der Flußwiderstand derart dimensioniert, daß bei der niedrigsten denkbaren Betriebstemperatur $T_{soll}$ der gewünschte Soll-Fluß $C_{soll}$ im kontinuierlichen Betrieb, d.h. ohne getaktete Betätigung des Quetschventils, eingehalten wird. Steigt die Temperatur auf $T_1$ an, bei der die Flußrate durch den Flußwiderstand doppelt so groß ist wie die der Temperatur $T_{soll}$, so wird das Quetschventil derart getaktet, daß es jeweils nur die Hälfte der Zeit geöffnet ist. Dadurch ergibt sich ein Gesamtfluß von $C_{soll}$.

Steigt die Temperatur weiter auf $T_2$ an, so ist die Flußrate durch den Flußwiderstand bei gleichbleibenden Druck $3 \cdot C_{soll}$. Das Quetschventil wird daher derart getaktet, daß das Ventil zu zwei Dritteln der Zeit geschlossen und zu einem Drittel der Zeit geöffnet ist. Dadurch, daß die Geschlossen-Zeit des Quetschventils doppelt so lang wie die Offen-Zeit des Quetschventils, in der ein Fluß von $3 \cdot C_{soll}$ durch den Flußwiderstand strömt, ist, ergibt sich wiederum ein Gesamtfluß von $C_{soll}$.

Eine zeitliche variable Dosierrate kann durch eine zusätzliche Beeinflussung des Puls-Pausen-Verhältnisses realisiert werden. Ferner kann mit Hilfe des Quetschventils eine vollständige Abschaltung des Flusses erreicht werden. Dieses Ventil verhindert bei einem Störfall, bei dem der Auslaßdruck den maximalen Druckbereich des Druckgebers übersteigt, einen Rückfluß in das Fluidreservoir sowie eine eventuell mögliche Diffusion von Fluid aus dem Reservoir.

Um die Genauigkeit der Einstellung der Flußrate zu verbessern, beziehungsweise um eine Dosierüberwachungsfunktion, die automatisch feststellt, ob ein Medikamentenfluß besteht und wie groß dieser ist, zu implementieren, kann das erfindungsgemäße Medikamenten-Dosiersystem ferner einen Drucksensor aufweisen, der vor dem Flußwiderstand angeordnet ist, um den Druck, mit dem das flüssige Medikament beaufschlagt ist, zu erfassen. Bei der Steuerung des Puls-Pausenverhältnisses zum Einstellen der Flußrate wird dann nicht der Wert des konstanten Drucks, der durch den Druckgeber geliefert wird, sondern der Wert des Drucks, der durch den Drucksensor erfaßt wird, verwendet. Dadurch können Schwankungen des Drucks, die durch den Druckgeber oder andere Systemparameter bedingt sein können, ausgeglichen werden. Alternativ zu einem Drucksensor kann das System auch zwei Drucksensoren aufweisen, wobei einer der Drucksensoren vor dem Flußwiderstand angeordnet ist, während der andere Drucksensor hinter dem Flußwiderstand angeordnet ist. Bei der Ermittlung des Puls-Pausen-Verhältnisses über den Zusammenhang zwischen der Flußrate und der Viskosität des Mediums, des effektiven Strömungsquerschnitts und der Länge des Flußwiderstandes sowie des auf das Medium wirkenden Drucks, wird dann statt des Druckwerts, den der Konstantdruckgeber liefert, die Druckdifferenz zwischen dem Einlaß des Flußwiderstandes und dem Auslaß des Flußwiderstandes verwendet.

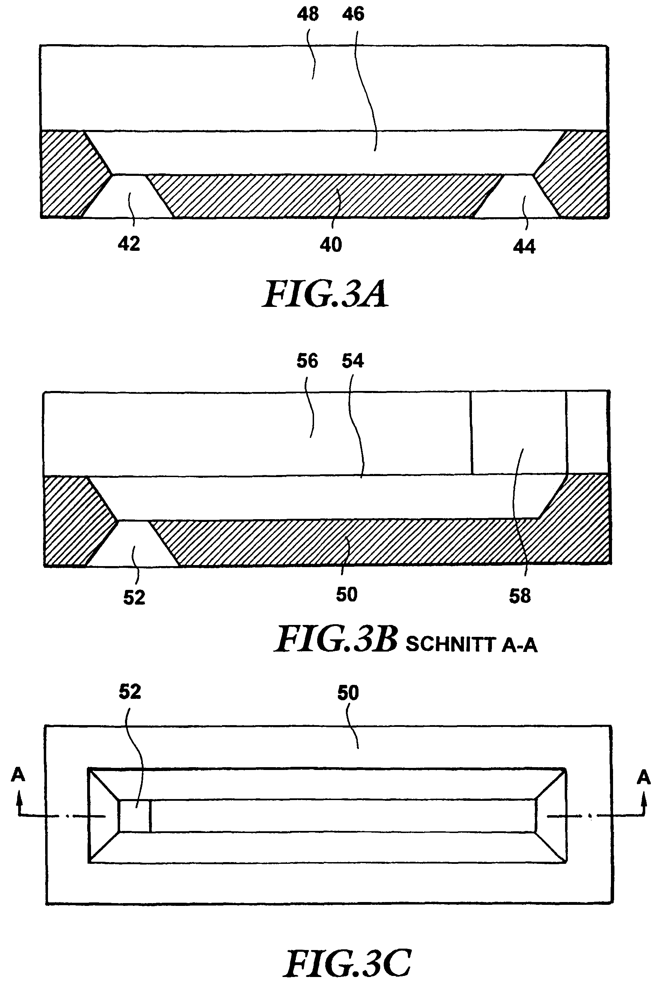

In Fig. 3A ist ein Ausführungsbeispiel eines mikromechanisch gebildeten Flußwiderstandes, der bei der vorliegenden Erfindung verwendbar ist, dargestellt. In einer Hauptoberfläche eines Substrats 40 sind eine Einlaßöffnung 42 und eine Auslaßöffnung 44 gebildet. In der anderen Hauptoberfläche des Substrats 40 ist ein Strömungskanal 46 derart gebildet, daß die Einlaßöffnung 42 und die Auslaßöffnung 44 mit dem Strömungskanal 46 fluidmäßig verbunden sind. Die Einlaß-und die Auslaßöffnung sind als planare Strukturen in dem Substrat realisiert. Das Substrat 40 kann beispielsweise ein Halbleiter-Chip, z.B. ein Silizium-Chip, sein, in dem beliebige Querschnitte und Anordnungen, z.B. eine Mäanderform, des Kanals, unter Berücksichtigung des gewünschten Strömungswiderstandes, gebildet sind, wobei beliebige naßchemische Verfahren, z.B. KOH-Ätzen oder trockenchmische Verfahren, z.B. Plasma-Ätzen verwendet werden können. Alternativ kann das Substrat 40 ein mittels bekannter mikromechanischer Einspritzverfahren hergestelltes Kunststoffsubstrat sein.

Auf der Oberfläche des Substrats 40, in der der Kanal 46 gebildet ist, ist eine Abdeckung 48 angebracht. Die Abdeckung 48 und die Ausnehmung 46 in dem Substrat 40 definieren den Querschnitt des Flußwiderstandes. Die Abdeckung kann beispielsweise ein Glasdeckel, der in einem anodischen Bondverfahren aufgebracht wird, sein. Alternativ kann ein weiteres Halbleitersubstrat, beispielsweise aus Silizium, als Abdeckung 48 verwendet werden.

In den Fig. 3B und 3 C ist eine Schnittansicht bzw. eine Draufsicht ohne Abdeckung eines weiteren Ausführungsbeispiels eines mikromechanisch gefertigten Flußwiderstandes dargestellt. In einem Substrat 50 ist eine Einlaßöffnung 52 und ein Strömungskanal 54 gebildet. Über dem Strömungskanal 54 ist wiederum eine Abdeckung 56 angebracht. Bei diesem Ausführungsbeispiel ist die Auslaßöffnung 58 jedoch in der Abdeckung 56 angebracht. Die Materialien und die Herstellungsverfahren des Substrats und der Abdeckung können denen des bezüglich Fig. 3A beschriebenen Ausführungsbeispiels entsprechen.

Fig. 3C zeigt eine Draufsicht der in Fig. 3B dargestellten Schnittansicht, wobei Fig. 3B einen Schnitt entlang der Linie A-A von Fig. 3C darstellt. Die in den Figuren dargestellten trapezförmigen Öffnungen können durch herkömmliche Ätzverfahren erhalten werden. Die Abhängigkeit der Flußrate von einem solchen trapezförmigen Querschnitt ist in der Technik bekannt, siehe die oben genannte Schrift "Micro Channels for Applications and Liquid Dosing and Flow Rate Measurement".

Der Temperatursensor des erfindungsgemäßen Medikamenten-Dosiersystems ist vorzugsweise in den oben beschriebenen mikromechanischen Aufbau integriert.

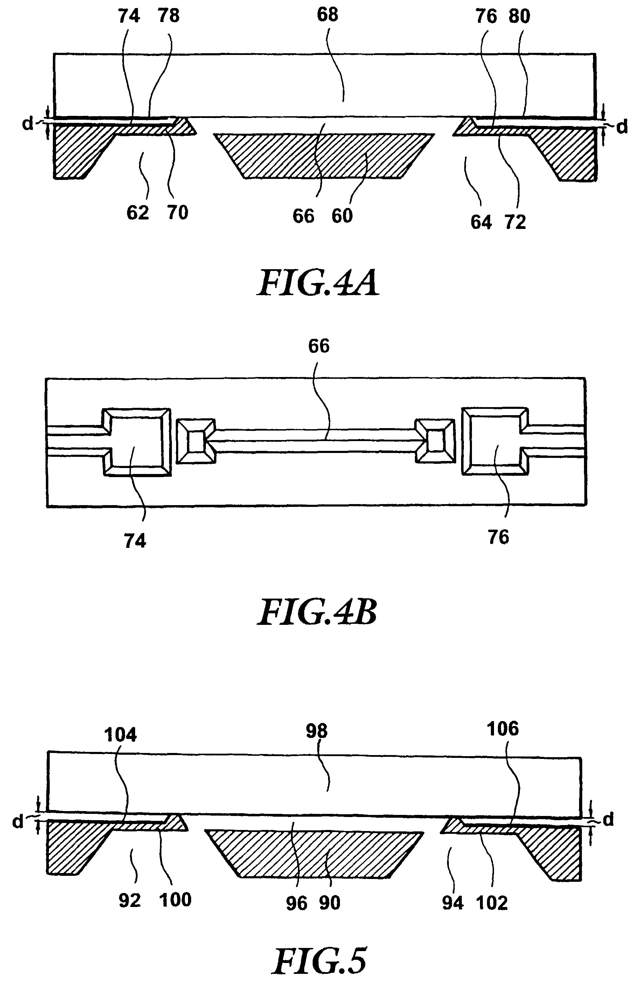

Die Fig. 4A und 4B zeigen eine Queschnittansicht bzw. eine Draufsicht eines weiteren Ausführungsbeispiels eines mikromechanisch gefertigten Flußwiderstandes, bei dem Drucksensoren am Einlaß und am Auslaß des Flußwiderstandes integriert sind, um eine Messung des Differenzdrucks zu ermöglichen. In einer Hauptoberfläche eines Substrats 60 sind eine Einlaßöffnung 62 und eine Auslaßöffnung 64 gebildet. In der anderen Hauptoberfläche des Substrats 60 ist ein Strömungskanal 66 gebildet, der mit der Einlaßöffnung 62 und der Auslaßöffnung 64 fluidmäßig verbunden ist. Der Strömungskanal 66 definiert zusammen mit einer Abdeckung 68 den Querschnitt des Flußwiderstandes.

Das in den Fig. 4A und 4B dargestellte Ausführungsbeispiel weist ferner zwei kapazitive Drucksensoren auf, die durch auf Membranen 70 und 72 angeordnete Membranelektroden 74 und 76 sowie gegenüberliegend auf der Abdeckung angeordnete Elektroden 78 und 80 gebildet sind. Die Membranelektroden 74 und 76 sind jeweils um den Abstand d von den Gegenelektroden 78 und 80 beabstandet. Bei Druckschwankungen verformt sich jeweils die auf der Membran angebrachte Membranelektrode, wodurch die Kapazität der Elektrodenanordnung als Ausgangssignal verändert wird.

Fig. 5 zeigt ein weiteres Ausführungsbeispiel eines mikromechanisch gefertigten Flußwiderstandes, der bei der vorliegenden Erfindung verwendet werden kann. Wiederum sind in unterschiedlichen Hauptoberflächen eines Substrats eine Einlaßöffnung 92, eine Auslaßöffnung 94 sowie ein Strömungskanal 96 derart gebildet, daß die Einlaßöffnung 92 und die Auslaßöffnung 94 mit dem Strömungskanal 96 jeweils fluidmäßig verbunden sind. Der Strömungskanal 96 ist an der Oberseite durch eine Abdeckung 98 abgedeckt. Wiederum sind in dem Substrat 90 Membranen 100 und 102 gebildet, auf denen bei dem in Fig. 5 dargestellten Ausführungsbeispiel piezoresistive Widerstände 104 und 106 integriert sind. Der Wert der piezoresistiven Widerstände 104 und 106 ändert sich abhängig von der mechanischen Verformung der Membran und kann als Meßgröße für den Druck ausgewertet werden. Die piezoresistiven Elektroden 104 und 106 sind um einen Bewegungsabstand d von der Unterkante der Abdeckung 98 beabstandet.

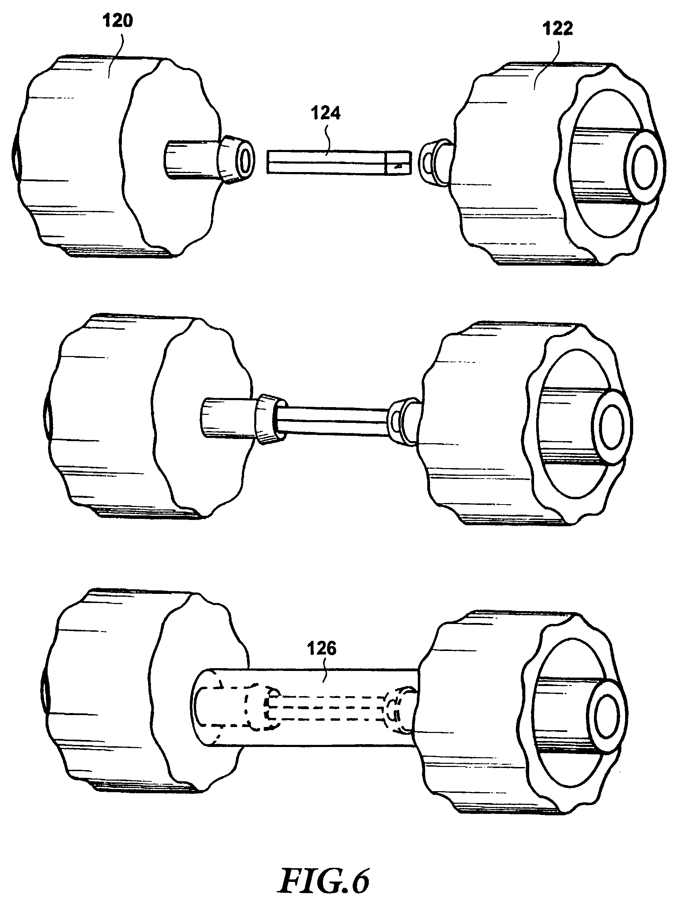

Zusätzlich zu den oben beschriebenen mikromechanisch gebildeten Flußwiderständen kann auch das Fluidreservoir sowie die Verbindung zwischen dem Fluidreservoir und dem Flußwiderstand bei dem Medikamenten-Dosiersystem gemäß der vorliegenden Erfindung in einem Substrat gebildet sein, vorzugsweise in dem gleichen Substrat wie der Flußwiderstand. Ein derart strukturiertes Substrat kann in einem Gehäuse gehäust werden, um den Anschluß eines Druckgebers sowie der Schlaucheinrichtung an das Fluidreservoir bzw. den Flußwiderstand zu ermöglichen. Ein Beispiel zur fluidischen Ankopplung von mikromechanisch gefertigten Flußwiderständen, das Kompatibilitätsanforderungen mit bereits bestehenden Standards erfüllt, ist in Fig. 6 dargestellt.

Das in Fig. 6 dargestellte System basiert auf sogenannten Luer-Verbindern bekannt. Das in Fig. 6 dargestellte Luer-System ist durch ein zweiteiliges Gehäuse mit einem ersten Gehäuseteil 120 und einem zweiten Gehäuseteil 122 realisiert. Das Gehäuseteil 120 und das Gehäuseteil 122 weisen geeignete Verbindungselemente und interne Fluidkanäle auf, die für die Einlaß- und Auslaßgeometrie des mikromechanisch gefertigten Fluidsystems passend sind. Das mikromechanisch gefertigte Fluidsystem 124 wird dann auf diese Einlaß- und Auslaß-Öffnungen durch ein dichtendes Montageverfahrens aufgesetzt. Dieses Aufsetzen kann beispielsweise durch Kleben oder eine Montage mit O-Ringen erfolgen. Abschließend kann eine äußere Umhüllung 126 des mikromechanischen Fluidsystems vorgesehen werden. Derartige Anschlußsysteme für mikromechanisch gefertigte Fluidführungsvorrichtungen sind in der Technik bekannt.

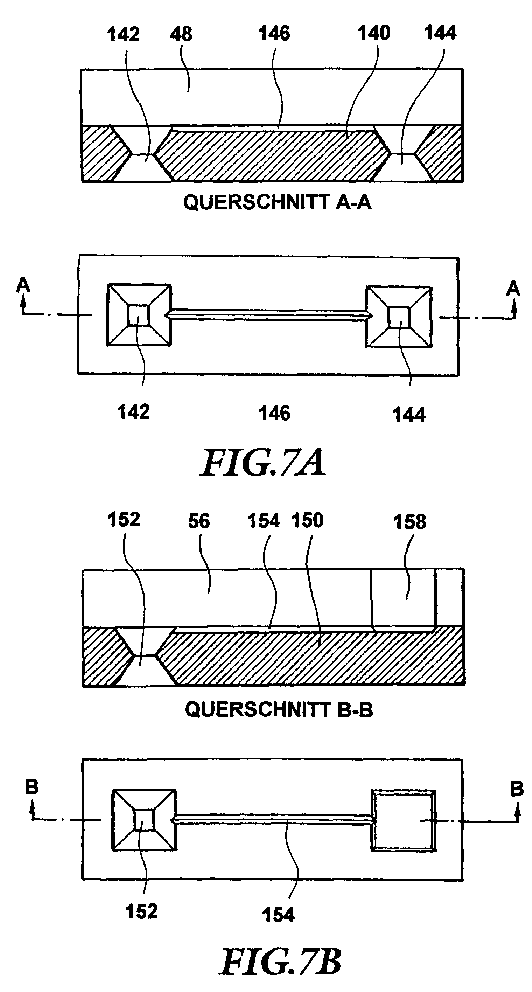

In den Fig. 7A und 7B sind zwei weitere Ausführungsbeispiele von Flußwiderständen, die in dem erfindungsgemäßen System verwendet werden können, dargestellt.

Bei dem in Fig. 7A dargestellten Ausführungsbeispiel sind zwei Durchlaßöffnungen, eine Einlaßöffnung 142 und eine Auslaßöffnung 144, durch ein Substrat 140 gebildet. Die Durchlaßöffnungen können beispielsweise durch das Ätzen von beiden Hauptoberflächen eines Halbleiter-Substrats, das beispielsweise aus Silizium besteht, her in demselben gebildet sein. In fluidmäßiger Verbindung mit den Durchlaßöffnungen ist ein, beispielsweise V-förmiger, Kanal 146 in einer Hauptoberfläche des Substrats 140 gebildet. Auf der Hauptoberfläche des Substrats 140, in der der Kanal 146 gebildet ist, ist wiederum die Abdeckvorrichtung 48 angeordnet. Dadurch definieren wiederum der Kanal und die Abdeckvorrichtung die Flußrestriktion des Flußwiderstands. Bei diesem Ausführungsbeispiel des Flußwiderstands ist der Mikrokanal 146 in unterschiedlicher Tiefe zu den Durchlaßöffnungen 142 und 144 auf einer Hauptoberfläche des Substrats angeordnet.

Fig. 7B zeigt eine Querschnittansicht bzw. eine Draufsicht eines weiteren Ausführungsbeispiels eines mikromechanisch gefertigten Flußwiderstands, der bei dem erfindungsgemäßen System verwendet werden kann. Bei dem in Fig. 7B dargestellten Ausführungsbeispiel ist jedoch im Gegensatz zu dem Ausführungsbeispiel von Fig. 7A eine der Durchlaßöffnungen, die Auslaßöffnung 158 in der Abdeckvorrichtung 56 angeordnet. Die Einlaßöffnung 152 und der Kanal 154 sind in gleichartiger Weise wie bei dem in Fig. 7A dargestellten Ausführungsbeispiel in einem Substrat 150 ausgebildet.

Die vorliegende Erfindung schafft somit ein Medikamenten-Dosiersystem, das die kontinuierliche Steuerung der Flußrate durch das getaktete Betätigen eines Quetschventils ermöglicht, jedoch eine kostengünstige Wegwerflösung bietet, da das Quetschventil nicht mit Fluid in Berührung kommt und somit ein dauerhaftes Bauteil ist.

Die Steuervorrichtung des erfindungsgemäßen Medikamenten-Dosiersystems kann neben dem Steuern des Puls-Pausen-Verhältnisses des Quetschventils aufgrund der gemessenen Temperatur weitere Funktionen durchführen. Beispielsweise kann mittels derselben eine patientengesteuerte Gabe einer einstellbaren Dosis eines Medikaments, z.B. die Bolus-Gabe eines Schmerzmittels im akuten Notfall, beispielsweise bei einem Schmerzanfall, durch die Eingabevorrichtung, die eine Bolus-Taste aufweisen kann, erfolgen. Ferner ist eine Dosierüberwachung realisierbar, beispielsweise durch eine Einhaltung von Zeitsperren nach der Bolus-Gabe, um eine zu häufige Bolus-Gabe und damit eine Suchtgefahr zu verhindern. Die Steuereinheit ermöglicht es ferner, die Anzahl der patientengesteuerten Bolus-Gaben, aktuelle Dosierraten und eventuell auftretende Systemstörungen aufzuzeichnen, um zusätzliche Informationen über das Patientenverhalten zu gewinnen. Die Auslesung derartiger Daten kann beispielsweise über eine integrierte Datenschnittstelle erfolgen. Ferner sind Alarmfunktionen, beispielsweise ein Warnsignal bei einer Unterdosierung oder einer Gerätefehlfunktion implementierbar. Über die integrierte Datenschnittstelle ist ferner eine beliebige Programmierbarkeit verfügbar, d.h. beliebige Vorgaben von Dosierprofilen, Bolus-Konzentrationen und Refraktär-Zeiten können programmiert werden.

## Patentansprüche

1. Medikamenten-Dosiersystem, bestehend aus einer auswechselbaren Einheit und einer dauerhaften Einheit, wobei die auswechselbare Einheit folgende Merkmale aufweist:

    ein Fluidreservoir (20) zur Aufnahme eines unter Druck setzbaren, flüssigen Medikaments;

    einen Temperatursensor (28) zum Erfassen der Temperatur des flüssigen Medikaments;

    einen mit einem Flußwiderstand (22) versehenen, fluidmäßig mit dem Fluidreservoir (20) verbundenen Fluidkanal; und

    eine mit dem Fluidkanal (24) verbundene Schlaucheinrichtung (30); und

    wobei die dauerhafte Einrichtung folgende Merkmale aufweist:

    eine Quetschventilvorrichtung (32) zum Zusammenquetschen der Schlaucheinrichtung (30); und

    eine Steuereinrichtung (34), die mit dem Temperatursensor (28) und der Quetschventilvorrichtung (32) gekoppelt ist, um eine Flußrate des flüssigen Medikaments durch ein getaktetes Betätigen der Quetschventilvorrichtung (32) abhängig von der erfaßten Temperatur zu steuern.

2. Medikamenten-Dosiersystem, bestehend aus einer auswechselbaren Einheit und einer dauerhaften Einheit, wobei die auswechselbare Einheit folgende Merkmale aufweist:

    ein Fluidreservoir (20) zur Aufnahme eines unter Druck setzbaren, flüssigen Medikaments;

    einen mit einem Flußwiderstand (22) versehenen, fluidmäßig mit dem Fluidreservoir (20) verbundenen Fluidkanal; und

    eine mit dem Fluidkanal (24) verbundene Schlaucheinrichtung (30); und

    wobei die dauerhafte Einrichtung folgende Merkmale aufweist:

    einen Temperatursensor zum Erfassen der Temperatur des flüssigen Medikaments;

    eine Quetschventilvorrichtung (32) zum Zusammenquetschen der Schlaucheinrichtung (30); und

    eine Steuereinrichtung (34), die mit dem Temperatursensor (28) und der Quetschventilvorrichtung (32) gekoppelt ist, um eine Flußrate des flüssigen Medikaments durch ein getaktetes Betätigen der Quetschventilvorrichtung (32) abhängig von der erfaßten Temperatur zu steuern.

3. Medikamenten-Dosiersystem gemäß Anspruch 1 oder 2, das ferner einen Konstantdruckgeber (36) aufweist, um das flüssige Medikament vor dem Flußwiderstand (22) mit einem konstanten Druck zu beaufschlagen.

4. Medikamenten-Dosiersystem gemäß einem der Ansprüche 1 bis 3, das ferner zumindest einen Drucksensor (72, 76, 80; 70, 74, 78) zum Erfassen des Drucks des flüssigen Medikaments vor dem Flußwiderstand (22) aufweist, wobei der zumindest eine Drucksensor mit der Steuereinrichtung (34) gekoppelt ist, und wobei die Steuereinrichtung (34) die Flußrate des flüssigen Medikaments abhängig von der erfaßten Temperatur und dem erfaßten Druck steuert.

5. Medikamenten-Dosiersystem gemäß Anspruch 4, das ferner einen weiteren Drucksensor zum Erfassen des Drucks des flüssigen Medikaments nach dem Flußwiderstand aufweist, wobei der weitere Drucksensor mit der Steuereinrichtung (34) gekoppelt ist, und wobei die Steuereinrichtung (34) die Flußrate des flüssigen Medikaments abhängig von der erfaßten Temperatur und der Differenz der beiden erfaßten Drücke steuert.

6. Medikamenten-Dosiersystem gemäß Anspruch 5, bei dem die Steuereinrichtung eine Datenschnittstelle, eine Eingabevorrichtung und eine Anzeigevorrichtung aufweist.

7. Medikamenten-Dosiersystem gemäß einem der Ansprüche 1 bis 6, bei dem zwischen dem Fluidreservoir (20) und dem den Flußwiderstand (22) aufweisenden Fluidkanal (24) ein Filter (26) angeordnet ist.

8. Medikamenten-Dosiersystem gemäß einem der Ansprüche 1 bis 7, bei dem der Flußwiderstand mittels mikromechanischer Herstellungsverfahren als eine mikromechanische Struktur gebildet ist.

9. Medikamenten-Dosiersystem gemäß Anspruch 7, bei dem der Temperatursensor und/oder die Drucksensoren integriert mit dem Flußwiderstand als mikromechanische Struktur realisiert sind.

10. Medikamenten-Dosiersystem gemäß Anspruch 8 oder 9, bei dem die mikromechanische Struktur aus Silizium gebildet ist.

**Claims**

1. A medicament dosing system comprising a replaceable unit and a permanent unit, the replaceable unit having the following features:

    a fluid reservoir (20) for receiving therein a pressurizable liquid medicament;

    a temperature sensor (28) for detecting the temperature of said liquid medicament;

    a fluid channel which is provided with a flow resistor (22) and which is in flow communication with the fluid reservoir (20); and

    a hose means (30) which is connected to the fluid channel (24); and

    the permanent unit having the following features:

    a squeezing valve means (32) for squeezing the hose means (30) together; and

    a control means (34) which is coupled to the temperature sensor (28) and the squeezing valve means (32) so as to control a flow rate of the liquid medicament by clocked actuation of said squeezing valve means (32) depending on the temperature detected.

2. A medicament dosing system comprising a replaceable unit and a permanent unit, the replaceable unit having the following features:

    a fluid reservoir (20) for receiving therein a pressurizable liquid medicament;

    a fluid channel which is provided with a flow resistor (22) and which is in flow communication with the fluid reservoir (20); and

    a hose means (30) which is connected to the fluid channel (24); and

    the permanent unit having the following features:

    a temperature sensor for detecting the temperature of said liquid medicament;

    a squeezing valve means (32) for squeezing the hose means (30) together; and

    a control means (34) which is coupled to the temperature sensor (28) and the squeezing valve means (32) so as to control a flow rate of the liquid medicament by clocked actuation of said squeezing valve means (32) depending on the temperature detected.

3. A medicament dosing system according to claim 1 or 2, comprising in addition a constant pressure transmitter (36) for applying a constant pressure to the liquid medicament before the flow resistor (22).

4. A medicament dosing system according to one of the claims 1 to 3, comprising in addition at least one pressure sensor (72, 76, 80; 70, 74, 78) for detecting the pressure of the liquid medicament before the flow resistor (22), the at least one pressure sensor being coupled to the control means (34), and the control means (34) controlling the flow rate of the liquid medicament depending on the temperature detected and the pressure detected.

5. A medicament dosing system according to claim 4, comprising in addition a further pressure sensor for detecting the pressure of the liquid medicament after the flow resistor, said further pressure sensor being coupled to the control means (34), and said control means (34) controlling the flow rate of the liquid medicament depending on the temperature detected and the difference between the pressures detected.

6. A medicament dosing system according to claim 5, wherein the control means comprises a data interface, an input device and a display device.

7. A medicament dosing system according to one of the claims 1 to 6, wherein a filter (26) is arranged between the fluid reservoir (20) and the fluid channel (24) provided with the flow resistor (22).

8. A medicament dosing system according to one of the claims 1 to 7, wherein the flow resistor is formed by means of micromechanical production methods as a micromechanical structure.

9. A medicament dosing system according to claim 7, wherein the temperature sensor and/or the pressure sensors are implemented together with the flow resistor as a micromechanical structure.

10. A medicament dosing system according to claim 8 or 9, wherein the micromechanical structure consists of silicon.

**Revendications**

1. Système de dosage de médicaments, composé d'une unité échangeable et d'une unité permanente, l'unité échangeable présentant les caractéristiques suivantes :

   un réservoir à fluide (20) destiné à recevoir un médicament liquide pouvant être mis sous pression ;
   un capteur de température (28) destiné à capter la température du médicament liquide ;
   un canal à fluide, muni d'une résistance d'écoulement (22), en communication de fluide avec le réservoir à fluide (20) ; et
   un dispositif flexible (30) relié au canal à fluide (24) ; et
   le dispositif permanent présentant les caractéristiques suivantes :
   un dispositif soupape de compression (32), destiné à comprimer le dispositif flexible (30) ; et
   un dispositif de réglage (34) couplé au capteur de température (28) et au dispositif soupape de compression (32), destiné à régler une quantité d'écoulement du médicament liquide par une commande en séquence du dispositif soupape de compression (32), en fonction de la température captée.

2. Système de dosage de médicaments, composé d'une unité échangeable et d'une unité permanente, l'unité échangeable présentant les caractéristiques suivantes :

   un réservoir à fluide (20) destiné à recevoir un médicament liquide pouvant être mis sous pression ;
   un canal à fluide, muni d'une résistance d'écoulement (22), en communication de fluide avec le réservoir à fluide (20) ; et
   un dispositif flexible (30) relié au canal à fluide (24) ; et
   le dispositif permanent présentant les caractéristiques suivantes :
   un capteur de température destiné à capter la température du médicament liquide ;
   un dispositif soupape de compression (32), destiné à comprimer le dispositif flexible (30) ; et
   un dispositif de réglage (34) couplé au capteur de température (28) et au dispositif soupape de compression (32), destiné à régler une quantité d'écoulement du médicament liquide par une commande en séquence du dispositif soupape de compression (32), en fonction de la température captée.

3. Système de dosage de médicaments suivant la revendication 1 ou 2, présentant, par ailleurs, un capteur de pression constante (36), destiné à soumettre le médicament liquide, avant la résistance d'écoulement (22), à une pression constante.

**4.** Système de dosage de médicaments suivant l'une des revendications 1 à 3, présentant, par ailleurs, au moins un capteur de pression (72, 76, 80 ; 70, 74, 78), destiné à capter la pression du médicament liquide avant la résistance d'écoulement, l'au moins un capteur de pression étant couplé au dispositif de réglage (34), et le dispositif de réglage (34) réglant la quantité d'écoulement du médicament liquide en fonction de la température captée et de la pression captée.

**5.** Système de dosage de médicaments suivant la revendication 4, présentant, par ailleurs, un autre capteur de pression, destiné à capter la pression du médicament liquide après la résistance d'écoulement, l'autre capteur de pression étant couplé au dispositif de réglage (34), et le dispositif de réglage (34) réglant la quantité d'écoulement du médicament liquide en fonction de la température captée et de la différence entre les deux pressions captées.

**6.** Système de dosage de médicaments suivant la revendication 5, dans lequel le dispositif de réglage présente une interface de données, un dispositif d'entrée et un dispositif d'affichage.

**7.** Système de dosage de médicaments suivant l'une des revendications 1 à 6, dans lequel, entre le réservoir à fluide (20) et le canal à fluide (24) présentant la résistance d'écoulement (22), est disposé un filtre (26).

**8.** Système de dosage de médicaments suivant l'une des revendications 1 à 7, dans lequel la résistance d'écoulement est réalisée, à l'aide de procédés de fabrication micromécaniques, sous forme de structure micromécanique.

**9.** Système de dosage de médicaments suivant la revendication 7, dans lequel le capteur de température et/ou les capteurs de pression sont réalisés sous forme de structure micromécanique, intégrés avec la résistance d'écoulement.

**10.** Système de dosage de médicaments suivant la revendication 8 ou 9, dans lequel la structure micromécanique est réalisée en silicium.

**36**　　**20**　　**26**　**24**　**22**　　　**30**　**32**

**28**

**34**

# FIG.1

*FIG.2*

48  46

**FIG.3A**

42  40  44

56  54

**FIG.3B** SCHNITT A-A

52  50  58

52  50

A  A

**FIG.3C**

74  78                    68                    76  80

d                                                              d

62  70              66  60              64  72

*FIG.4A*

66

74                    76

*FIG.4B*

104                    98                    106

d                                                              d

92  100              96  90              94  102

*FIG.5*

14

**FIG.6**

142    48    146    140    144

QUERSCHNITT A-A

A — A

142    146    144

*FIG.7A*

152    56    154  150    158

QUERSCHNITT B-B

B — B

152    154

*FIG.7B*

*FIG.8*